# EUROPEAN PATENT SPECIFICATION

(11) **EP 0 529 074 B1**
(45) Date of publication and mention of the grant of the patent: **21.08.1996**
(21) Application number: 90907451.0
(22) Date of filing: 16.05.1990
(51) Int. Cl.: G01N 25/04, G01N 25/06, G01N 33/20

(54) **METHOD OF JUDGING CARBON EQUIVALENT, CARBON CONTENT, AND SILICON CONTENT OF CAST IRON AND ESTIMATING PHYSICAL AND MECHANICAL PROPERTIES THEREOF, AND COOLING CURVE MEASURING CUP USED FOR SAID METHOD**
VERFAHREN ZUR BEURTEILUNG DES KOHLENSTOFFEQUIVALENTS, DES KOHLENSTOFFGEHALTES UND DES SILIZIUMGEHALTES IN GUSSEISEN UND ABSCHÄTZUNG DER PHYSIKALISCHEN UND MECHANISCHEN EIGENSCHAFTEN SOWIE ABKÜHLKURVENMESSTOPF FÜR DIESES VERFAHREN
PROCEDE D'EVALUATION DE L'EQUIVALENT-CARBONE, DE LA TENEUR EN CARBONE ET DE LA TENEUR EN SILICIUM DE FONTE DE FER ET D'EVALUATION DE SES PROPRIETES PHYSIQUES ET MECANIQUES, AINSI QUE GODET DE MESURE DE LA COURBE DE REFROIDISSEMENT UTILISE POUR METTRE EN OEUVRE CE PROCEDE

(43) Date of publication of application: 03.03.1993
(73) Proprietor: METEC CORPORATION, Sumida-ku, Tokyo 131 (JP)
(72) Inventor: YAMAGUCHI, Takeshi, 10-13, Mukojima 2-chome, Tokyo 131 (JP)
(74) Representative: Pendlebury, Anthony
(86) International application number: JP9000617
(87) International publication number: WO9118285

(56) References cited:
- DE-A- 2 733 341
- JP-A-54 036 797
- JP-B-50 036 199
- JP-B-54 011 718
- JP-B-58 007 186

## Description

### Technical Field

This invention relates to methods of accurately measuring a cooling curve of molten cast iron by thermal analysis thereby determining the carbon equivalent, carbon content and silicon content of the iron and estimating the physical and mechanical properties of the iron whereby the furnace front administration of the pre-casting process in the casting factory is sufficiently administered for a cupola and the melt charging from the furnace is measured, and a cooling curve measuring cup used in these methods.

### Background Technique

The principle for the measurement of carbon equivalent is such of monitoring, as of pseudobinary system, originally the elements of iron, carbon and silicon basically of ternary system at the initial thermal arrest temperature (primaly crystal to be liquidus) when the melt of cast iron solidifies thereby to make it carbon equivalent. Though carbon equivalent is expressed in various ways the most general application thereof is to define it as the total percent of carbon plus one-third (Si% plus P%). There is a further application in which eutectic temperature is measured, interrelation of the temperature with liquid, liquid plus solid, and solid is reviewed thereby to determine the silicon and carbon contents, and the physical and mechanical properties of the cast iron can be estimated on the basis, as function, of the time until solidification of the melt. It is known that according to the application, the numerical values thus obtained are corrected and analyzed depending on the modification of practical profile, material and the like of the casting, it is possible to know the state of the melt accurately and more quickly and field-like than any other analyzing method by means of a cup which is manufactured by a known technique and pre-arranged before the pouring of the melt to allow the melt to be administered before casting, and in case the constituents and various properties of the molten cast iron differ from those intended a suitable pretreatment can be carried out. These known techniques are disclosed in US-A-3,267,732 and JP-A-820,206 based thereon, but neither of them can sufficiently achieve the object.

Compared with the time when said known techniques were proposed the casting materials are selected in a greater range in the present casting industries. High technology has now been desired as compacted graphite iron (so-called CV cast iron) or austempered ductile iron (so-called ADI cast iron) has appeared and cast iron alloys have been improved and developed whereby a technique of administering the in-situ furnace front melt is required correctly and more quickly. That is, iron and carbon system is a binary system whereas iron, carbon and silicon system is a ternary system. In binary system the eutectic temperature is constant whereas in the ternary system it is maximum or minimum, and conditions have become diverse such that cast irons are greatly affected by the silicon content, and according to the additive elements for cast iron alloys the eutectic temperature rises for some elements and is lowered for some others. A further complicated phenomenon is that in the solidification of the basic elements iron, carbon and silicon of ternary system there are two equilibriums of completely stable solidification of iron-carbon (graphite)-silicon system and metastable solidification of iron-carbon (cementite)-silicon system.

Additionally, as the cooling rate changed or the additive elements differ the two equilibriums alternately occur in the same melt in some occasions. Such a multi-equilibrium problem causes the complication of solidification of cast iron system. Under the existing circumstances where operation by cupola is gradually shifting these days to the melting by electric furnace because of the control to the environmental contamination, serious problems are presented not only to steels but also to cast irons, as the problems not only for the characteristic change of the melt caused with time after melting and the simple change of the constituents of the melt but also for the oxygen content, oxide or solved oxygen content in the melt.

This makes known thermal analysis method unsatisfactory. It is therefore necessary to accurately measure not only the primaly crystalized temperature but also the eutectic temperature, so that, it is required to measure the cooling curve in a safe white pig iron state (iron and cementite system), and the measurement by semi-stable solidification becomes necessary, as requisite condition, not only for the cooling curve but also for samples for mechanical analysis such as in emission spectrum analysis method, X-ray analysis method or the like.

The inventor of this invention has made extensively his research in an attempt to solve the above problems. As a method of adding graphitization-hindering elements such as tellurium, bismuth and boron as metastable solidification promotors according to prior art there can be mentioned the one of adding, as a paint for chill wash or the like, metal tellurium powder or the like in a measuring cup, which method bases on the above-referred patent inventions. However, it is questionable in those inventions whether said elements are accurately added always in a constant proportion, and it is doubtful whether correct primaly crystalized and eutectic temperatures are always obtained from the samples of melts of cast irons. Referring to a method of obtaining a cooling curve JP-A-820,206 describes in its claims to add into the melt bismuth, boron, cerium, lead, magnesium and tellurium, and compounds and mixtures thereof as stabilizer. However, cerium and magnesium are spherification reaction agents and spherification stabilizers for typical ductile cast irons, and they disturb the equilibrium state of metal-stable solidification of iron-cementite, system to make the measurement of respective primaly crystalized and eutectic temperatures. It is naturally not until the measurement of the equilibrium state that the primaly crystalized and eutectic temperatures are measured. Cerium and magnesium necessarily pass through deoxidation, desulfuration and decarburization processes before the spherification of graphite, and it will be clear it is inconvenient to use elements which obtain a decarburization action, in measuring the carbon equivalent and carbon content.

### Disclosure of the Invention

In the present invention various studies and experiments have been carried out to remove the above demerits of the known techniques. The technical constitution of the invention lies in a method of measuring a cooling curve by means of thermal analysis of molten cast iron, in which onto the inner surface of a cooling curve measuring cup there is fixed a compressed powder moulding or sintered moulding prepared of a metallic powdery body of tellurium, bismuth, boron, zinc or aluminium or a mixture thereof a melt of cast iron is poured into said measuring cup when primaly crystalized and eutectic temperatures based on the metastable solidification of iron, cementite and silicon clearly appear on the cooling curve of the cast iron whereby the carbon equivalent, carbon content and silicon content of the iron are determined and also the physical and mechanical properties thereof are estimated. On the other hand, the present invention relates to a cooling curve measuring cup by means of thermal analysis of cast iron, characterized in that a compressed metallic powder moulding or sintered moulding of tellurium, bismuth, boron, zinc or aluminium or a mixture thereof is arranged at and fixed onto the inner bottom surface of said cup, while enclosing a thermocouple.

As described above, in view that a sufficient deoxidative effect is produced if used even in a small amount and the primaly crystalized and eutectic temperatures of cast iron are not reversely affected even remained as an alloy element, the inventor of this invention has paid his attention to aluminium. He has also noticed that by adding a small amount of zinc to enhance the effect of the metastable solidification promotors such as tellurium, bismuth and boron and to control a little exothermic phenomenon which occurs due to the deoxidation reaction of aluminium, it is possible to cancel the heat generation by the evaporation latent heat to allow the effect of the metastable solidification to be improved, and to completely remove the affection caused by the oxide or solved oxygen content. According to the invention, therefore, a powdery body of tellurium, bismuth or boron is mixed with that of aluminium, the mixture is formed as a compressed powder moulding or sintered moulding, and the moulding is fixed to the inside of the cooling curve measuring cup. The mixture as a moulding well reacts with the pouring melt of the cast iron so that it is possible to quickly and accurately measure the carbon equivalent, carbon content and silicon content of the iron and to determine the physical and mechanical properties of the cast iron.

### Brief Description of the Drawings

Fig. 1 is a plan view of a cooling curve measuring cup of the invention; and
Fig. 2 is a vertical sectional view taken along the line II-II of Fig. 1.

### Best Embodiment for Carrying out the Invention

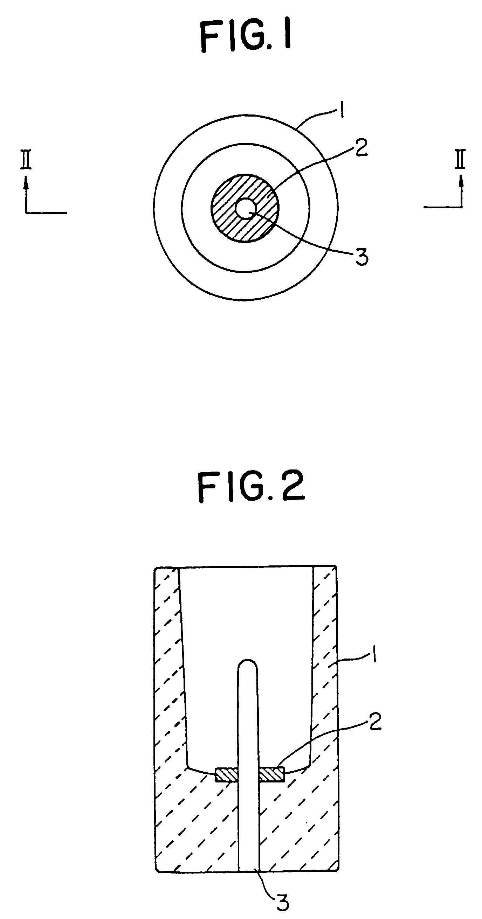

The method of the invention has been carried out by using a cooling curve measuring cup (1) shown in Figs. 1 and 2. A ring-like moulding (2) of the invention is arranged, while enclosing a thermocouple (3), at the bottom surface within said cup (1). Said moulding (2) is prepared in such a manner that metallic powder of tellurium, bismuth, boron, zinc or aluminium or a powdery mixture thereof is compressed or sintered for moulding. A suitable composition thereof may be in the following range because of the above reasons:

| | |
|---|---|
| Aluminium | 3 - 20% by weight |
| Zinc | 3 - 20% by weight |
| Tellurium | The rest |

Further, bismuth and boron can be substituted for part of said tellurium content up to 50% by weight.

It has been confirmed from the result of experiments that with a composition less than the above range it is incapable of achieving the desired object of the invention while with an additive amount exceeding the upper limit it is no more than a waste of expensive elements.

Additionally, the arrangement of the moulding (2) within the measuring cup, which is shown in the drawings, just shows the best embodiment, and the arrangement is not restricted to the one as shown in the drawings.

### Example 1

### Moulding composition

| | |
|---|---|
| Aluminium | 7.3% by weight |
| Zinc | 9.7% by weight |
| Tellurium | The rest |

### Conditions for moulding compressed powder

A powdery body of said composition was uniformly mixed, the mixed powder was charged in a powder metallurgical die having a desired size, and it was moulded to be a ring-like moulding of about 1 mm thickness by a powder metallurgical press. In the preparation of such a moulding, binder is not used at all for the powdery body, but it makes no hindrance to employ a small amount of a volatile binder according to the mixing proportion of the powder. As shown in Fig. 2, a ring-like moulding thus prepared is pressed-in to the bottom center of a measuring cup manufactured by shell moulding sand while making a pocket suited to the outside diameter and thickness of the moulding so as to be fixed there. Since naturally the inside diameter of the moulding is made a little larger than the outside diameter of a thermocouple protective pipe any trouble does not occur for inserting the thermocouple. Samples of cast irons to be measured, in various compositions, being at 1400 - 1405°C, were poured into the cups, and the following result was obtained.

| | Hypo-eutectic cast iron | | Hyper-eutectic cast iron | |
|---|---|---|---|---|
| | (1) | (2) | (1) | (2) |
| Proeutectic temperature | 1,169°C (2,136°F) | 1,154°C (2,110°F) | 1,131.5°C (2,069°F) | 1,111.5°C (2,033°F) |
| Eutectic temperature | 1,115°C (2,039°F) | 1,116°C (2,041°F) | 1,112.5°C (2,035°F) | 1,107°C (2,025°F) |
| Carbon equivalent | 4.17 | 4.30 | 4.48 | 4.71 |
| Carbon content | 3.52 | 3.66 | 3.78 | 3.91 |
| Silicon content | 1.94 | 1.89 | 2.08 | 2.39 |

### Example 2

| | |
|---|---|
| Aluminium | 6.94% by weight |
| Zinc | 9.72% by weight |
| Bismuth | 10.50% by weight |
| Tellurium | The rest |

A mixture of the above constituents was moulded in the same manner as in Example 1, and the same operation was effected by using the moulding when the result almost same as in Example 1 was obtained.

The function and effect of the present invention are as follows:
(1) Measurement can be made in a broad range from hypo-eutectic side to hyper-eutectic side and even to cast iron alloys.
(2) Different from conventional methods of coating chill wash or metallic powder of tellurium or the like to the inner surface of a measuring cup it is possible to correctly add a binder of predetermined composition. Unlike chill wash, cooling i.e. so-called recalescence often does not appear on the cooling curve, being sometimes great and sometimes small in the tellurium content in the respective measuring cups. Said recalescence enables the measurement to be difficult. Due to the development of the compound binder it is capable of positively grasping the lower limit value of ternary eutectic temperature thereby to know always the stable primaly crystalized and eutectic temperatures in the metastable solidification region of iron-cementite system.
(3) As a result, according to the invention, it is possible to achieve a sufficient object with only the additive amount 0.2 - 1.0% by weight of said compressed powder moulding or sintered moulding to said cast iron. As a result of the measurement it has become to be able to make an accurate measurement almost by 100% within the ranges of ±0.05% for carbon equivalent, ±0.05% for carbon content and ±0.15% even for silicon content. This results in demonstrating sufficiently the ability of the cup as a tool for administering the furnace front melt in field in a casting factory, and the invention will contribute to management of melting working to cope with advance and development of future casting technology.

### Industrially Possible Application

According to the present invention, a cooling curve can be accurately measured by thermal analysis of cast iron thereby determining the carbon equivalent, carbon content and silicon content in the iron and estimating the physical and mechanical properties of the iron whereby the furnace front administration of the pre-casting processes in a casting factory is easily effected. Additionally, an effective analyzing means in field is provided for distribution and delivery of the melt for from a blast furnace and for sorting of steel making pig iron and various kinds of pig irons for casting.

## Claims

1. A method of measuring a cooling curve by means of thermal analysis of cast iron, characterized in that a compressed powder moulding or sintered moulding made of a metallic powdery body of tellurium, bismuth, boron, zinc or aluminium or a mixture of said elements is fixed to the inner surface of a cup for said cooling curve measurement, a melt of cast iron is poured into said cup, and primaly crystalized and eutectic temperatures based on the metastable solidification of iron, cementite and silicon are clearly displayed on the cooling curve of the cast iron thereby to determine the carbon equivalent, carbon content and silicon content in the iron and to estimate the physical and mechanical properties of the iron.

2. A method of determing the carbon equivalent, carbon content and silicon content of cast iron and estimating the physical and mechanical properties of the iron as claimed in Claim 1 wherein the composition of said compressed powder moulding or sintered moulding is in the range of 3 - 20% by weight of aluminium, 3 - 20% by weight of zinc, and the rest of tellurium.

3. A method of determing the carbon equivalent, carbon content and silicon content of cast iron and estimating the physical and mechanical properties of the iron as claimed in Claim 1 wherein bismuth or boron is substituted for part of tellurium which is one of the constituents of said compressed powder moulding or sintered moulding.

4. A cup for measuring a cooling curve by means of thermal analysis of cast iron characterized in that a compressed metallic powder moulding or sintered moulding prepared from tellurium, bismuth, boron, zinc and/or aluminium is arranged at and fixed to the inner bottom surface of said cup, while enclosing a thermocouple.

## Patentansprüche

1. Verfahren zum Messen einer Abkühlungskurve vermittels einer thermischen Analyse von Gußeisen, dadurch gekennzeichnet, daß ein verdichtetes Preßpulverformteil oder ein gesinteres Formteil eines Metallpulverkörpers aus Tellur, Wismut, Bor, Zink oder Aluminium oder einer Mischung dieser Bestandteile an der inneren Oberfläche einer Pfanne für das Messen dieser Abkühlungskurve angeordnet ist, eine Schmelze aus Gußeisen in diese Pfanne gegossen wird und daß ursprünglich kristallisierte und eutektische Temperaturen, die auf der metastabilen Erstarrung von Eisen, Zementit und Silizium beruhen, klar auf der Abkühlungskurve des Gußeisens dargestellt sind und dadurch das Kohlenstoffequivalent bestimmen, den Kohlenstoffgehalt und den Siliziumgehalt im Eisen und die physikalischen und mechanischen Eigenschaften des Eisens abschätzen.

2. Verfahren zum Bestimmen des Kohlenstoffequivalents, des Kohlenstoffgehalts und des Siliziumgehalts von Gußeisen und zum Abschätzen der physikalischen und mechanischen Eigenschaften von Eisen nach Anspruch 1, dadurch gekennzeichnet, daß die Zusammensetzung des verdichteten Presspulverformteiles oder des gesinterten Formteiles im Bereich von 3 - 20 Gewichtsprozent Aluminium, 3 - 20% Gewichtsprozent Zink und der Rest aus Tellur ist.

3. Verfahren zum Bestimmen des Kohlenstoffequivalents, des Kohlenstoffgehalts und Siliziumgehalts von Gußeisen und Abschätzen der physikalischen und mechanischen Eigenschaften des Eisens nach Anspruch 1, dadurch gekennzeichnet, daß Wismut oder Bor zu einem Teil durch Tellur ersetzt wird, welches eines der Bestandteile des verdichteten Presspulverformteiles oder des gesinterten Formteiles ist.

4. Topf zum Messen einer Abkühlungskurve vermittels thermischer Analyse von Gußeisen, dadurch gekennzeichnet, daß ein Pressmetallpulverformteil oder Sinterformteil hergestellt aus Tellur, Wismut, Bor, Zink und/oder Aluminium vorgesehen ist und an der inneren Bodenoberfläche dieses Topfes festgemacht ist und ein Thermoelement beinhaltet.

## Revendications

1. Procédé de mesure d'une courbe de refroidissement par analyse thermique de fonte de fer, caractérisé en ce qu'un moulage de poudre comprimé ou un moulage fritté constitué d'un corps poudreux métallique de tellure, bismuth, bore, zinc ou aluminium ou d'un mélange de ces éléments est fixé à la surface interne d'un creuset de mesure de la courbe de refroidissement, un mélange de fonte de fer est versé dans le creuset et des températures initiales de cristallisation et d'eutectiques basées sur la solidification métastable du fer, de la cémentite et du silicium sont clairement affichées sur la courbe de refroidissement de la fonte de fer pour déterminer ainsi l'équivalent en carbone, le contenu en carbone et le contenu en silicium dans le fer et pour estimer les propriétés physique et mécanique du fer.

2. Procédé de détermination de l'équivalent en carbone, du contenu en carbone et du contenu en silicium de fonte de fer et d'estimation des propriétés physique et mécanique du fer selon la revendication 1, dans lequel la composition du moulage de poudre comprimé ou moulage fritté est dans la plage de 3 à 20 % en poids d'aluminium, de 3 à 20 % en poids de zinc et le reste en tellure.

3. Procédé de détermination de l'équivalent en carbone, du contenu en carbone et du contenu en silicium de fonte de fer et d'estimation des propriétés physique et mécanique du fer selon la revendication 1, dans lequel du bismuth ou du bore est substitué pour partie à du tellure qui est l'un des constituants du moulage en poudre comprimé ou du moulage fritté.

4. Creuset de mesure de la courbe de refroidissement par analyse thermique de fonte de fer, caractérisé en ce qu'un moulage de poudre métallique comprimé ou un moulage fritté préparé à partir de tellure, de bismuth, de bore, de zinc ou d'aluminium est disposé et fixé à la paroi interne inférieure du creuset, et enferme un thermocouple.
